# EUROPEAN PATENT SPECIFICATION

(11) **EP 3 504 965 B1**
(45) Date of publication and mention of the grant of the patent: **07.07.2021**
(21) Application number: 16778450.3
(22) Date of filing: 26.08.2016
(51) Int. Cl.: A01K 67/033, A01M 1/02, A01M 1/10, A01M 1/20

(54) **BIOLOGICAL PEST CONTROL DEVICE**
BIOLOGISCHES SCHÄDLINGSBEKÄMPFUNGS GERÄT
DISPOSITIF BIOLOGIQUE ANTI RAVAGEURS

(43) Date of publication of application: 03.07.2019
(73) Proprietor: Macadar Angier, Victor Alejandro, Montevideo (UY)
(72) Inventor: Macadar Angier, Victor Alejandro, Montevideo (UY)
(74) Representative: Durán-Corretjer, S.L.P.
(86) International application number: PCT/IB2016/055105
(87) International publication number: WO 2018/037265

(56) References cited:
- WO-A2-98/21934
- FR-A- 1 137 978
- US-A- 3 968 590
- US-A1- 2006 124 067

## Description

### TECHNICAL FIELD

The present invention relates to a biological pest control device, whose structure enables for it to be supported by a tree branch, or in an existing natural or artificial linear support in site, such as a wire, applied in agricultural and/or forestry production. With such a device, it is possible to free the contents of a capsule, wherein such contents can be substances and beneficial insects that help to control pests. Said substances may serve to repel pests from the zone, attract them and trap them inside the device, or attract them to the device to allow them to come into contact with toxic substances. With the proposed structure, auxiliary support fixing and low location of the center of gravity is achieved, and therefore decreased movement where it is located, which favors content protection of beneficial insects or substances against rain and wind with any speed or direction.

### STATE OF THE ART AND PROBLEMS TO BE SOLVED THEREOF

Among state of the art devices for biological pest control there are three distinct groups which are distinguished by the nature of the substance they release: a) devices that release beneficial insects, b) traps that release scents or other stimulants to attract pests to the device and poison them or get them trapped inside, and c) deterrents that release substances that repel the presence of pests in the area where the device is installed.

Among the first group, we can find devices that release beneficial insects which can be held by the plant branch by a suspending hook and which comprise a container, such as a cardboard box, pocket or bag inside which the beneficial element to be released is located. They are mainly used in closed crops, given that they are designed to be used in controlled environments, because they are not resistant to adverse weather conditions during prolonged periods of time. If used outdoors, the efficacy of the biological pest control strategy can be diminished.

There are other more complex devices which, given the nature of the beneficial insects, require special preparations to release the beneficial insects, such as disclosed in the Chinese document CN203618588U (Yin Yanqiong et al) of 4 June 2014, used to release parasitoid wasps. Such device is completely vulnerable to rain and has no protection against natural predators of the beneficial insects. Therefore, it is not suitable for outdoor use, given that leaving it outdoors may result in a decreased efficacy of the device, which also depends on adverse conditions and predators of the beneficial insects present in the area. Moreover, the device is designed to specifically work with one of the many existing parasitoids.

Other devices have feeding and protection areas against weather conditions, because they take into account beneficial insects which are to slow to hatch and emerge to the environment. It is worth mentioning the device disclosed in Chinese utility model CN203597253U (Ren We et al) published May 21, 2014. Such device has certain protection against the rain, but not against heavy wind storms where water can get inside from the side, nor does it have a firm anchoring against floods or winds. Its operation and dynamics of use are more complex and it must be arranged at the release zone, which increases the likelihood of failure due to human error.

Devices for releasing beneficial insects, especially the more complex ones, can have stability problems when suspended with a hook outdoors due to a pendulum effect which arises from suspending such devices from branches using hooks, wires or cords, higher than the one the same branch would experience, particularly when winds are strong, producing stirring of the device contents. This can cause alterations to the beneficial insects inside the device, depending on the species. If the device also features food substances, another drawback arises and it is the involuntary mixing of the device contents by these movements, as in the case of some of the devices which release parasitoid wasps with honey for their feeding. This can cause the eggs of beneficial insects to become stuck to their food, before the insects can hatch and go outside.

It is also known that such devices, as well as those of the subsequent categories, have efficacy problems given their water permeability and floodability when it is raining in a lateral direction due to strong winds.

This is the case of US3968590A which discloses an insect trap according to the precharacterising portion of claim 1. Such device is not prepared and has no protection of its content against heavy rain, as water can get inside through the big openings all along the perimeter side. Furthermore, US2006/124067A1, FR1137978A and WO98/21934A2 are also worth to be cited as state of the art.

In addition, though to a lesser degree, devices that are installed on the floor can result in vulnerability to flooding or the beneficial insects failing to achieve their goal given that they have to go through low areas with predators before they can reach the plants to fight pests; even more so, given that these devices are designed for use in controlled environments such as greenhouses, most of them are not prepared for the possible presence of water, which causes complications even when watering.

Additionally, none of these devices are designed for an easy arrangement and/or food placing, weather during manufacturing or in a laboratory prior to the release stage, which could avoid errors by the end user handling the device contents.

When appropriate, planning a feeding zone protected from outside dangers will ensure the beneficial insects will be more potent and even long-lasting to attack pests. The design of current devices does not feature an efficient safe zone for beneficial insects when they cannot go outside because of adverse weather conditions. These devices also lack a space for species who are born ready for reproduction to mate after feeding and before going outside. In some cases, this is necessary in order to ensure the establishment of pest control insect populations in the zone, which increases the efficiency and efficacy of the biological pest control strategy and reduces its cost.

Regarding the second group of biological pest control devices of the trap type, it is worth mentioning Chinese patent application CN1545380A (Knauf Werner et al) of November 10, 2004, which simulates being a round fruit to attract and trap or repel flying insects.

Patent US6860062B2 (Spragins), published March 1, 2005, discloses a simple device with limited protection against rain, which has a simple suspending mechanism via a cord, which causes the shaking of the contents when there is wind and the bending of the inlets. Because of that, its 360° rotary top is vulnerable to water entering through said inlets when it is raining and windy. The same thing happens to the devices of patents US 5057316 (Haim B. Gunner et al), October 15, 1991, and US6792713B2 (Erick Snell), September 21, 2004.

Additionally, said devices have problems with their range because, given that they are suspended by a hook, they need the target pest to be of the flying type. If it is a walking or crawling pest, they require the prey to walk along the wires or other suspending means, rendering any suspending means inefficient for insects with low mobility. Moreover, such traps are not always ready for use and must be assembled by the final user at the releasing zone, which increases the likelihood of human error because of a lack of experience and/or knowledge for carrying out the assembling.

The device disclosed in patent US 4400903 (Seidenberger), August 30, 1983, is designed in a smart way that takes advantage of the behavior of certain insects with marked phototropism. Its disadvantage, if one wants to use it with another types of pests, is that it is specifically limited to work with flying insects with positive phototropism. Another aspect to be considered when producing and assembling it is its complexity, given that it features several different materials.

360º convex or rotary tops of the state of the art are not appropriate for working with insects with marked geotropism because of the following: when a species that is going to be released (or trapped) has marked geotropism, it tends to move only in one way, preferably in the vertical direction, which makes it very unlikely that they would move alternately upwards and downwards to traverse from the inside to the outside of the device or vice versa, that is, alternatively switching vertical direction.

One factor to take into consideration is that there are insects with a marked negative geotropism, those who tend to stay away from the ground, so, if there is the need to design an exit path with a simple and natural trajectory, it must be vertical and upwards. Given that water goes in the opposite direction when falling and flowing, the device gets flooded when it rains or during watering.

Because of that, the present invention, as defined in claim 1, proposes a top part that offers maximum protection against adverse weather conditions for beneficial insects with both positive and negative geotropism, as well as neutral geotropism. With respect to these two devices, their exit is protected due to the fact that it faces an inner face of the top and it is above the level to which water can get accumulated, between a capsule and the top when water flows along the branch that goes through the device from side to side, so that water does not enter the capsule. Moreover, exit orifices are proximate to the branch of the affected plant, ensuring that the beneficial insects will be stimulated by the signals of the branch to go outside, immediately attacking the zone affected by pests.

Moreover, the proposed device serves as releasing device, trap or deterring device, depending on the contents. Contents are chosen according to the purpose of the device, and according to its function the position of the orifices in the pieces of the device is determined, and whether the container capsule used is composed by a single piece or two halves coupled together.

Other variables defined according to the species to be released or trapped are dimensions, opacity and thickness of the material. The different versions of the device are designed to work with any type of insect, even those with limited mobility, that cannot fly for a long time or that need access over the branch or very close to it, or with very marked behaviors (tropisms), such as positive or negative geotropism and/or phototropism. When the beneficial insects prefer to come out through the top part, the exit orifices are defined in the upper half of the container capsule and the eggs of beneficial insects are stored in the lower part. Further, eggs may be stored together with some substance and/or leaf cuttings in order to take advantage of the space and to handle them more comfortably. In the event that the species to be released prefers to come out through the lower part, the exit orifices are defined in the lower half of the container capsule and the eggs are placed in the upper half of said capsule.

In the present invention, installation actions are simplified obtaining better results than known devices, given that they require less actions. The user can install it in a very simple step of assembling the top and the capsule around the chosen branch or support. Moreover, the final user receives the capsule already prepared and assembled. This simple use increases efficacy, given that problems arising from human handling errors are minimized.

Devices disclosed in patents US6857579B2 (Harris Rano) del 22/02/2005, US20050199740A1 (Harris Rano), 15/SEP/2005, US20040083640A1 (Harris Rano), 06/MAY/2004, y US20040168363A1 (Baker Stanley), 2/SEP/2004, only serve to release scents, and they have the distinctive feature of been composed by a single piece fixed by wrapping around the branch, but in order to achieve this, the device must be made of flexible materials. In spite of their simplicity, this kind of fixing has the same problems as devices suspended by a hook, given that the contents get heavily stirred when there is wind, in addition to the natural movement of branches. Regarding use, in these devices the final user places the contents inside the capsule at the place of installation.

In the device of the present invention, morphology can be controlled, and its organic and smooth forms seek to integrate as an element of nature, without being literal, to work in different areas where leaves and plant types are different. Then, by selecting its color it is possible to make it stand out from the environment or mimic it and get lost in it.

In spite of its multiple uses, the design is easily customizable to adapt it to different needs in the biological pest control strategy or even to carry out field research. Its form can be easily changed without substantially altering costs or production times, given that it is possible to obtain the device by 3D printing.

Regarding its use, the device of the invention comes pre-prepared, which simplifies as much as possible its use and prevents problems arising from mishandling.

Finally, in the third group of biological pests control devices, deterrents, we find the device disclosed in U.S. patent US20130200172A1 (Opal Downer), published August 8, 2013, intended for dispelling pests by using chemical substances or by its form and colors. This device has disadvantages for customization as it does not enable easy production variation in order to change material parameters such as forms and sizes based on the pest to be repelled. When producing by plastic injection, large-scale serial production is required in order to recover investment costs.

The solution proposed by the present invention can be produced by traditional methods and it is also designed to be easily and readily 3D printed, e.g., by fused deposition modeling (FDM) techniques. This makes possible the variation of volumetric and material parameters to make them more specific and efficient for the pest to be attacked or repelled, and can be produced in batches of a few units and still be cost-effective.

Unlike devices for biological pests control of the state of the art of these three groups mentioned above, the device of the present invention comprises a top or "upper body" whose lower face is substantially concave, which is positioned above a capsule container or "container body" whose upper face is formed as a concave face, defining by its engagement with the upper body a cavity able to receive along the same, the positioning of a longitudinal part of the branch or other existing linear media in the installation site.

The proposed device is designed to be readily manufactured and to the desired extent, preferably with biodegradable materials, such as PLA polymer, so it is not necessary to collect the same from the field after use, with the advantage of economy logistics and simplifying of the application dynamics that this implies, in addition to the ecological advantages. This makes the invention sustainable and viable, because it allows to highly decrease costs of the biological pests control system, saving on traveling costs of operators, transportation of materials for disposal in a dumping site or recycling plant, and consequently work hours, given that operating costs of labor and transfer of materials or people are split.

Accessibility is crucial in these devices, given that there are very few which feature an exit or entrance near the transit zone of the insects. Many times the beneficial insects are small wasps and aerial mobility is very limited due to their small dimensions, because of that an exit right next to the plant is desirable, such as the one of the device of the invention.

Moreover, natural circulation is achieved, given that the space between the suspending means of the device and the branch enables free transit of the beneficial insects or other animals that move along the branch, without the need of climbing to the top of the device to cross to the other side, because cavity "C" has a vertical diameter that is larger than the diameter of the branch.

Beneficial insects are more comfortable given that they are not expelled from the device due to sudden movement, especially when there are adverse weather conditions. The device emulates natural movements by mimicking the movement of the branch and, because of that, the beneficial insects come out when they are prepared and exterior conditions are favorable.

Inside the device there is feeding zone protected from outside dangers which ensures the beneficial insects will be more potent and even long-lasting to attack pests. Moreover, said space serves as a meeting point where species who are born ready for reproduction can mate after feeding and before going outside. In some cases, this is necessary in order to ensure the establishment of pest control insect populations in the zone, which increases the efficiency of the strategy of the biological pest control device and reduces its cost.

The device maximizes the efficiency of releasing living load or attracting it, given that for a pest to access the device, or a beneficial insect to come out of it, it is not required for it to alternately switch between upward and downward direction, which makes it difficult or decreases efficiency, depending on the animals used, especially if they have well marked geotropism and tend only to go upwards, or to only go downwards. Being at an elevation is also beneficial, given that releasing at ground level is dangerous if the water level rises and the receiving chamber gets flooded.

Regarding the use as releasing device, it has a wider range of application than the prior art because in addition to the above advantages it also works with beneficial insects and pests with less mobility than those usually at ground level, for example, crawling or walking insects moving through the branches, that is, without the ability to fly or reduced flying capabilities such as small parasitoid wasps.

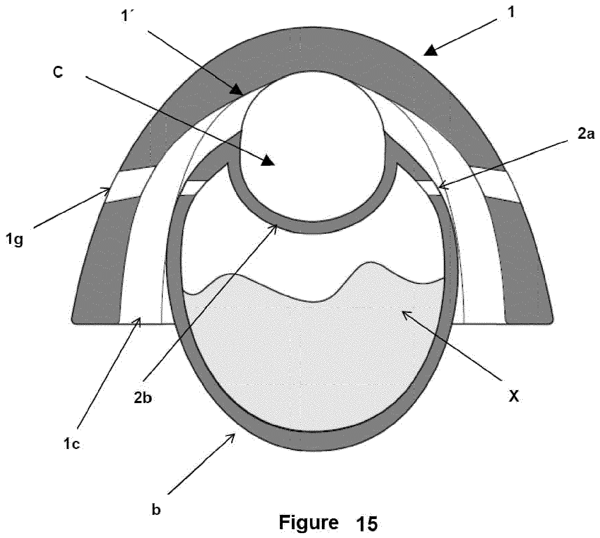

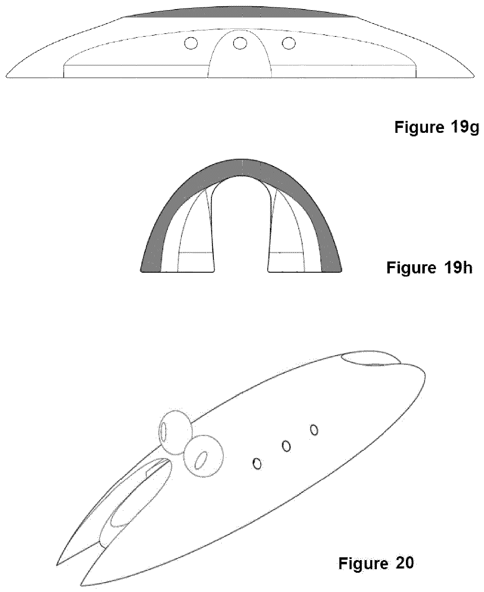

Regarding repelling pests, the device offers a maximum degree of customization, because it can be easily given any specific color or shape. Shapes may be 3D printed over the top part, as will be appreciated in figure 20, being the technical aspect of operation of the device similar to the trap shown in figure 15, in which a scent is released, except that the release substance serves to attract instead of repel.

On the other hand, keeping the arrangement of the parts, the volume can take any desired shape. In the figures are shown examples in which the external geometry has an ovoid profile. This is one of the most appropriate shapes for three reasons: a) When 3D printing is used, it is faster to produce these continuous forms than other types of forms, because the printer is required only to produce spiraling growing contours without a filling for the capsule part; b) Aerodynamic shapes offer minimal wind resistance, and c) it integrates with the rest of the organic forms of the environment.

### BRIEF DESCRIPTION OF THE INVENTION

The present invention discloses a biological pest control device according to claim 1.

The biological pests control device of the present invention essentially comprises two basic parts: a top part and a container capsule, and both achieve a firm and secure fixing to the support.

The top part or upper body protects the device against the rain and winds in any direction, thanks to its 360° enveloping profile, and below it a first cavity is defined which goes through the body from side to side, which will receive therein the branch that serves as support of the device on the plant, or another selected support, for example, a wire of a fence. This cavity is high enough, so that, in addition to receiving the branch, it enables rain to flow through and circulation of animals in both directions of the branch without touching the device.

The container capsule, below and coupled to the top part, has a second upper cavity or groove in the upper part of it which serves to couple the capsule to the interior part of the top, with a male-female coupling, in order to hold it. This container capsule has first orifices that allow scents, substance and/or insects to pass through, to enter or leave the capsule, and immediately move to the branch. Said container capsule allows the housing of the contents to be released, which may be insects, scents and/or other chemicals which may be toxic.

When the device is used to release scents, chemicals and/or toxics, the container capsule can be composed of a single piece.

If the device will be used to release beneficial insects, the container capsule is composed of three parts: one half that serves as container chamber, the other half that is the anteroom of the exit for the beneficial insects, and a grid that is located within the lower half of the capsule to separate areas.

The container or container chamber of beneficial insects within the container capsule is the half that houses the beneficial insects, even if they are still inside the egg. Light entering said chamber can be regulated by the opacity of the selected material and the thickness of the walls. Said regulation serves to prevent excessive heating due to a greenhouse effect.

The anteroom of the exit can be used, if necessary, as a meeting point for the beneficial insects to mate and reproduce before emerging to the environment. Said compartment has exit orifices to the environment that also serve as a filter that prevents natural enemies of the beneficial insects that are larger than the orifices from entering. It also features a partition wall that serves as a support means for food.

Finally, a grid or intermediate body with orifices along it divides the container chamber of the anteroom of the exit, which generates two well defined areas, one where unhatched beneficial insects are located and another where the insects come after hatching to feed, meet, mate and come out the device.

The grid prevents the eggs from entering the anteroom of the exit and sticking to food with the solution due to movement during transport of the device and environmental vibrations.

Said intermediate body or grid is placed to serve as a cover in the highest part of the lower half of the capsule or "lower hollow body". The upper half of the capsule or "upper grooved body" has a structural partition wall under the cavity in which the branch is received, which can hold food and also helps support the grid inside the lower hollow body and prevents it from entering the upper grooved body. When the upper grooved body is used as anteroom of the exit and feeding zone, said structural partition wall is used to place the food.

As can be appreciated, the particular dimensions of the device can be scaled and modified; because of that, its factory size is selected, for example, based on the amount of content to be released or the size of the supporting branch.

Because the device can release different types of contents, the parts of the capsule can adapt based on the characteristics of the contents to be released so that the capsule as a whole achieves its functionality. In the case of using the device with beneficial insects with negative geotropism, they tend to get away from the ground moving in the upward direction, therefore the upper grooved body will have exit orifices and will serve as anteroom of the exit and feeding zone, and the lower hollow body serves as container for the eggs of the beneficial insects. In the case of using the device with beneficial insects with positive geotropism, these insects travel in the opposite direction as the ones above, because of that the lower hollow body will have the exit orifices and will serve as anteroom of the exit, and the upper grooved body will serve as container chamber for beneficial insects.

All of the parts of the device can be manufactured using the same material and do not require any additional material, such as, for example, adhesives, which simplifies and reduces the cost of the logistics and assembly of the production process.

All different variants of the biological pest control device can be made of a biodegradable plastic material, for example, PLA, obtained from an organic substrate. If made of industrial compost, it takes only 90 days to biodegrade. If made of lower organic substrate and at low temperatures it takes more time to degrade, but it degrades nonetheless, transforming into biomass under the effect of hydrolysis and certain enzymes existing in the environment. Because of that, it is not necessary to collect the device from the field after use, with the advantage of economy logistics and simplifying of the application dynamics that this implies. In addition to being an ecological solution, it is sustainable and more advantageous, because it allows to highly decrease the overall costs of the biological control system, while increasing efficiency.

### BRIEF DESCRIPTION OF THE DRAWINGS

In order to make the object of the invention more understandable, figures illustrating preferred forms or variants of the present invention are shown, wherein:
Figure 1 is a first partial and complete exploded view of the biological pest control device variant used with beneficial insects with negative or neutral geotropism;
Figure 2 is a second exploded view of the device for use with beneficial insects with negative or neutral geotropism shown in figure 1;
Figures 3a and 3b are top and lower perspective views of the device assembled for use with beneficial insects with negative or neutral geotropism shown in figure 1;
Figure 4 depicts a cross-sectional view of the device for use with beneficial insects with negative or neutral geotropism shown in the preceding figures;
Figure 5a is a perspective view of the container capsule of the biological pest control device for beneficial insects with negative or neutral geotropism;
Figures 5b and 5c are respective top and lower perspective views of the top part of the biological pest control device shown in the preceding figures;
Figures 5d, 5e, 5f, 5g depict respective lower, lateral, front and top views of the top part shown in figures 5b y 5c;
Figure 5h shows the cross-section along line C1-C1 according to figure 5g;
Figure 5i shows the cross-section along line C2-C2 according to figure 5g;
Figures 6a and 6b are respective top and lower perspective views of the lower hollow body and container of beneficial insects of the container capsule, of the biological pest control device shown in the preceding figures;
Figures 6c, 6d, 6e depict respective lower, lateral and top views of the lower hollow body and container shown in figures 6a y 6b;
Figure 6f shows the cross-section along line C3-C3 according to figure 6c;
Figure 6g shows the cross-section along line C4-C4 according to figure 6c;
Figures 7a and 7b are respective top and lower perspective views of the upper grooved body or anteroom and feeding zone that form part of the container capsule of the biological pest control device shown in the preceding figures;
Figure 7c shows a lower view of the upper grooved body shown in figures 7a y 7b, with an enlarged cutout of the orifices 2a;
Figures 7d, 7e, 7f depict respective top, lateral and front views of the upper grooved body shown in figures 7a y 7b;
Figure 7g shows the cross-section along line C3-C3 according to figure 7c;
Figure 7h shows the cross-section along line C5-C5 according to figure 7e;
Figures 8a, 8b, 8c and 8d depict respective top, lower, lateral and front perspective views of the grid of the biological pest control device for beneficial insects with negative or neutral geotropism;
Figure 9 depicts a cross-sectional view of a biological pest control device variant for use with beneficial insects with positive or neutral geotropism;
Figure 10 is a perspective exploded view of the device for beneficial insects with positive or neutral geotropism shown in figure 9;
Figures 11a and 11b are perspective views of the top part and the container capsule of the biological pest control device for beneficial insects with positive or neutral geotropism shown in figure 9;
Figure 12 is a perspective view of the container capsule of the biological pest control device shown in figure 9;
Figures 13a and 13b are respective top and lower perspective views of the upper grooved body or container chamber of beneficial insects that form part of the container capsule shown in figure 12;
Figures 13c, 13d, 13e and 13f depict respective lower, lateral, top and front views of the chamber shown in figures 13a and 13b;
Figure 13g shows the cross-section along line C3-C3 according to figure 13c;
Figure 13h shows the cross-section along line C5-C5 according to figure 13d;
Figures 14a and 14b are respective top and lower perspective views of the lower hollow body or anteroom of the exit that forms part of the container capsule shown in figure 12, for use in a biological pest control device for beneficial insects with positive or neutral geotropism;
Figures 14c, 14d, 14e and 14f depict respective lower, lateral, top and front views of the anteroom of the exit shown in figure 14a;
Figure 14g shows the cross-section along line C3-C3 according to figure 14c;
Figure 14h shows the cross-section along line C4-C4 according to figure 14c;
Figure 15 is a cross-sectional view of a scent releasing device used as a trap or repellent for pests according to another embodiment of the present invention;
Figures 16a and 16b are top and lower perspective views of the scent releasing device used as a trap or repellent for pests shown in figure 15;
Figure 17 is a perspective view of the top part of the scent releasing device shown in figure 15;
Figures 18a and 18b are respective top and lower perspective views of the container capsule of the scent releasing device shown in figure 15;
Figures 18c, 18d, 18e depict respective top, frontal and lower perspective views of the container capsule of the biological pest control device shown in figure 15;

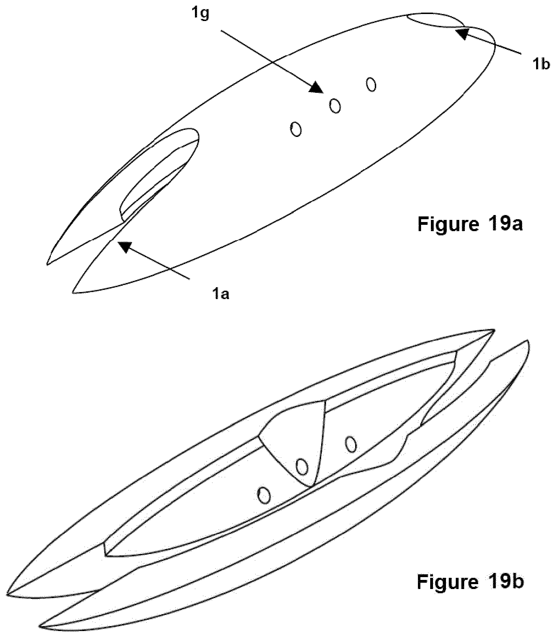

Figures 19a and 19b depict top and lower perspective views of the top part of the biological pest control device of the type shown in figure 15;

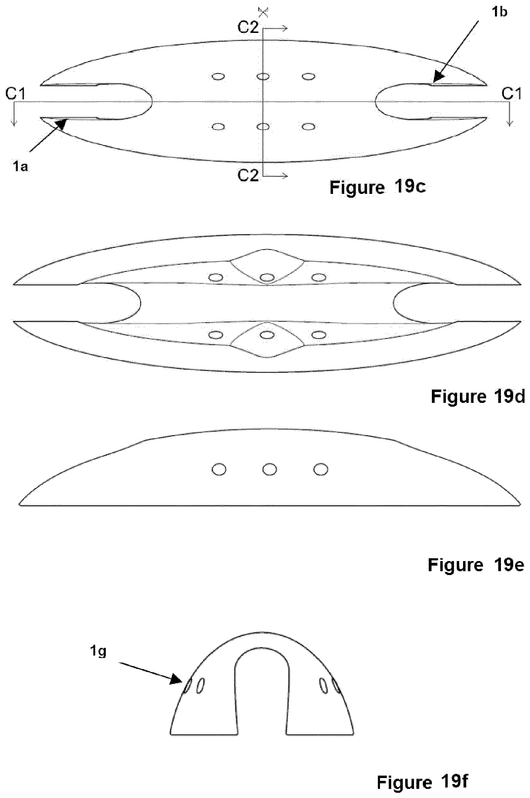

Figures 19c, 19d, 19e, 19f depict respective top, lower, lateral and front views of the top part shown in figures 19a y 19b;
Figure 19g shows the cross-section along line C1-C1 according to figure 19c;
Figure 19h shows the cross-section along line C2-C2 according to figure 19c;
Figure 20 is a perspective view of a scent releasing device, such as the one shown in figure 15, which incorporates superficial aesthetic elements to achieve a better pest repelling;

In all figures, like numerals and references indicate like elements.

### DETAILED DESCRIPTION OF THE INVENTION:

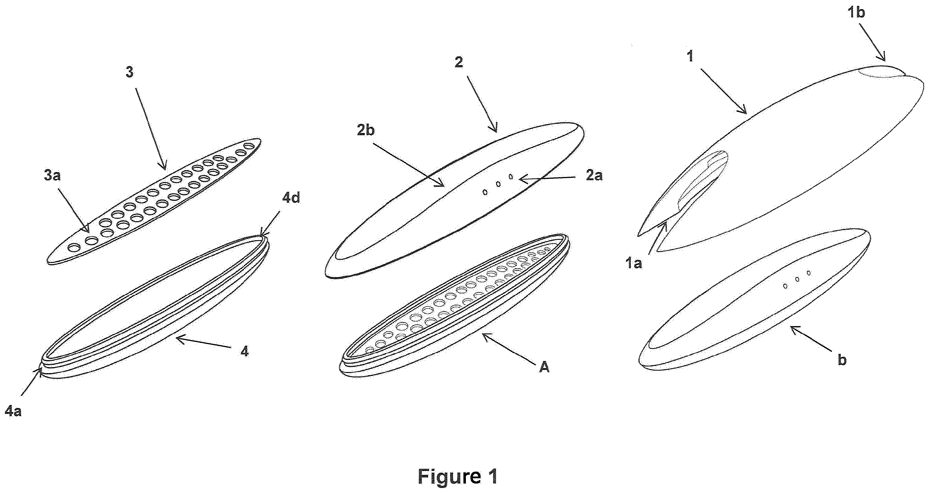

It can be seen in figure 1 that the biological pest control device for beneficial insects with negative or neutral geotropism comprises substantially ovoid bodies, particularly a top part 1 with recesses or grooves on ends 1a and 1b mounted on a container capsule "b". Moreover, said container capsule "b" comprises an upper grooved body 2 with side orifices 2a for the exit of beneficial insects, over which there is formed an elongated channel or concave surface 2b.

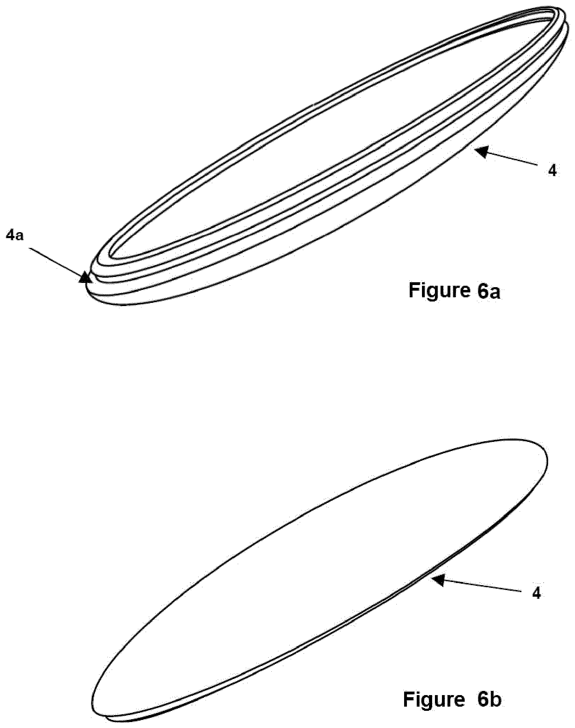

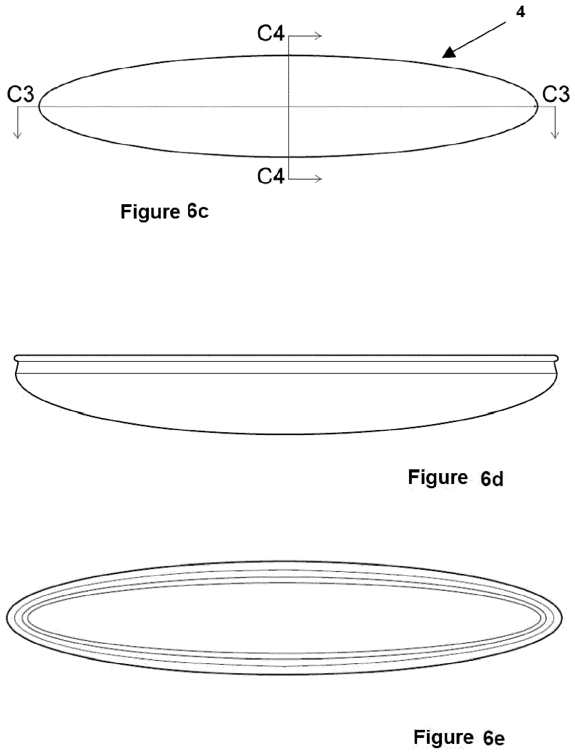

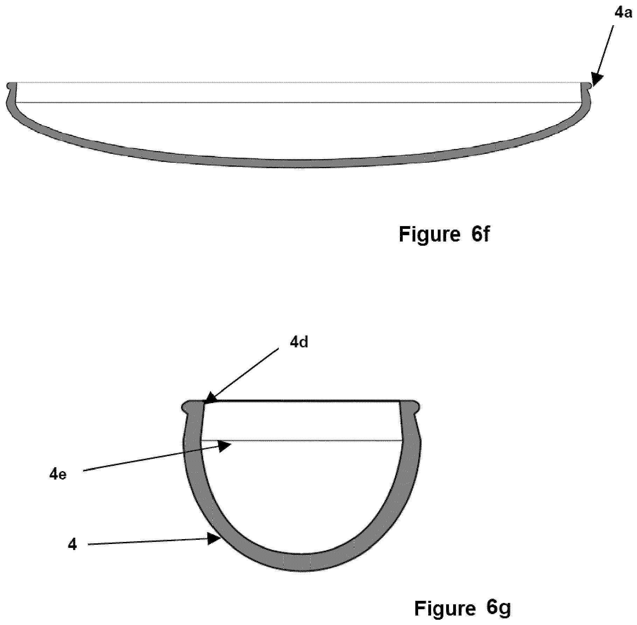

There is a grid 3, which is slightly flexible and then it can be bent, housed within said upper grooved body 2 and a lower hollow body 4 that consists of a bevel 4a for coupling. Figure 6f shows a generic bevel 4a for coupling. Grid 3 with orifices 3a allows for keeping leaf cuttings with eggs separated from food, which prevents the eggs from sticking to food or spreading and keeps them inside the container chamber for beneficial insects of container capsule "b".

Said grid 3 covers the lower hollow body 4 and has orifices 3a along designed so the grid can be easily unmounted with common sharp objects, such as a pen or pliers. Moreover, said grid 3 keeps the eggs of beneficial insects in the desired place, keeping the intended path for them to the feeding zone and outside clean.

The lower hollow body 4 enables housing the content to be released and it defines, on its perimeter edges, so as to snap on and lock to the upper grooved body 2, closing the container capsule "b".

Both halves of the capsule are coupled together and said closing can be used with any other type of suitable coupling, such as, for example, the bevel system of the capsule disclosed in patent US4866952A or any closing system with a security lock of the state of the art.

When assembling the device with beneficial insects, grid 3 is bent and introduced into the upper part of the cavity of the lower hollow body 4, this is due to the fact that the grid has a larger length than the perimeter inner borders 4d of said lower hollow body 4, forming a subset "A", over which the upper grooved body 2 is coupled, thus forming the container capsule "b". Finally, the top part 1 covers and couples to said container capsule "b".

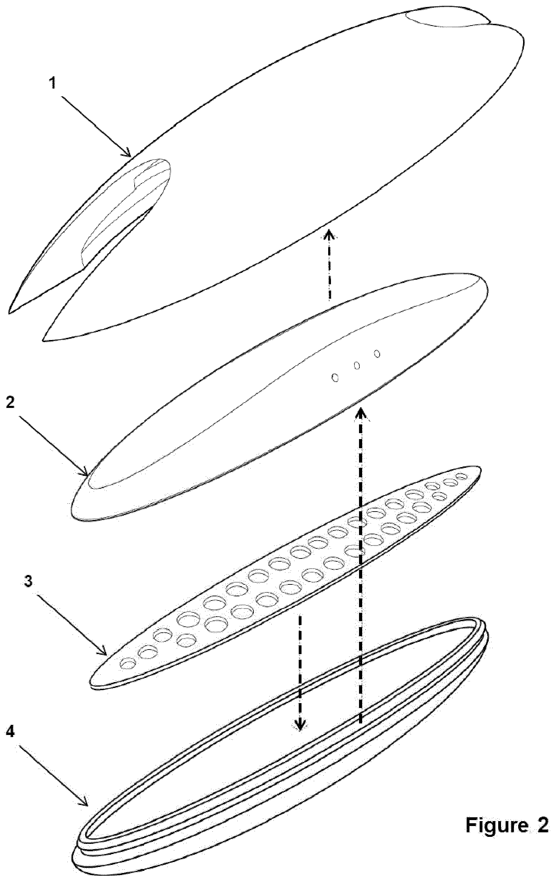

In figure 2 it can be seen the way in which the biological pest control device is assembled, fixing the grid 3 by bending pressure inside the lower hollow body 4, then enclosing with the upper grooved body 2, to form container capsule b, and finally coupling to the top part 1.

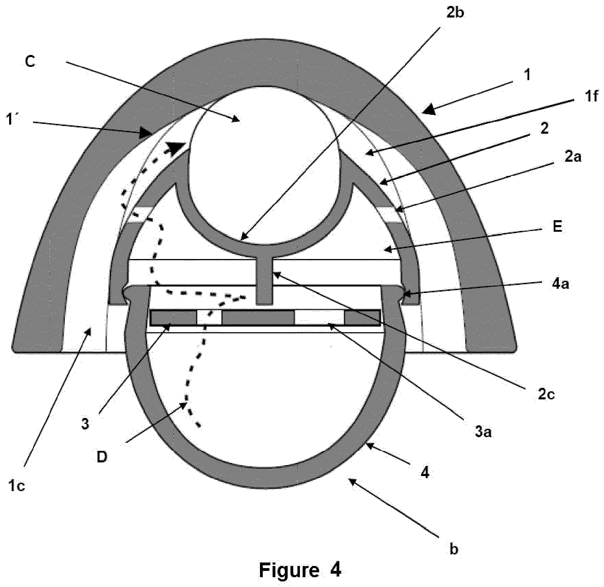

It can be seen in the cross-section of figure 4, the way in which a gap or cavity "C" is form, between the top part 1 and the container capsule "b" in order to readily position the tree branch in which it is desired to control a pest. As the device is coupled along the branch, it will mimic the natural movement of oscillation of the branch.

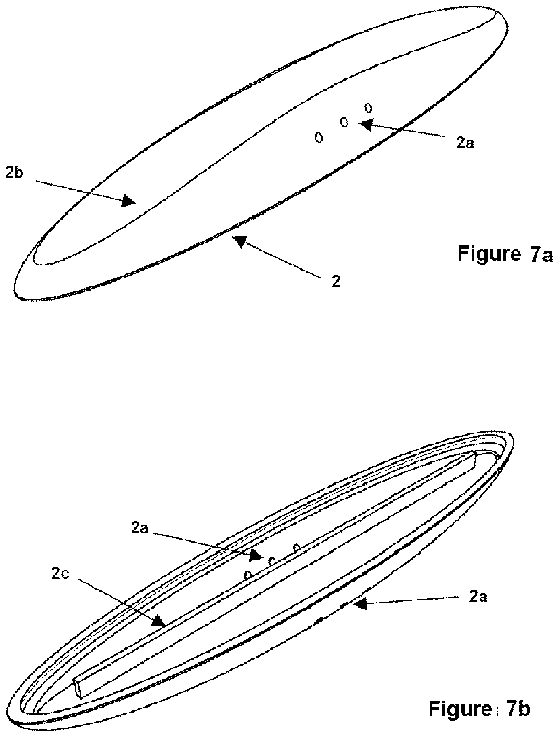

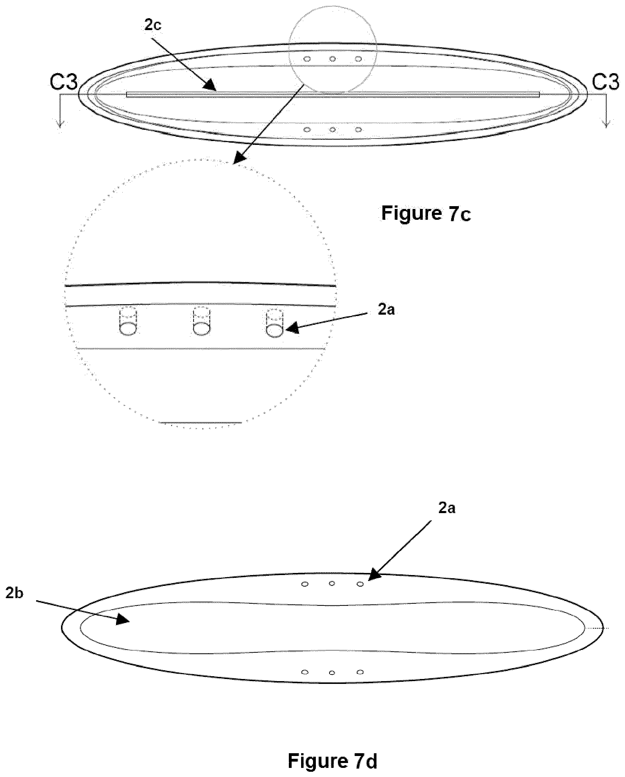

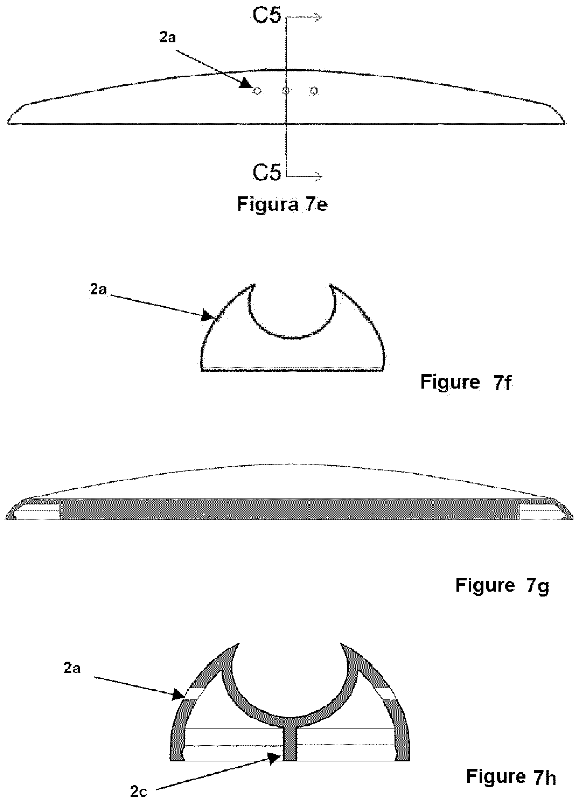

In figure 4, the expected path for the beneficial insects is indicated by "D", that is, the path from where they are born, in the lower hollow body 4, going through orifices 3a of grid 3 towards chamber "E" which serves as anteroom of the exit and feeding zone, wherein beneficial insects can mate and/or feed from the solution placed in the partition wall 2c (see also figure 7b); then they pass through orifices 2a of the upper grooved body 2, to reach cavity "C" where the branch is located, passing to the branch easily due to its proximity. These exit orifices 2a will be large enough for the beneficial insects to come out and move in any direction, but small enough to act as a barrier against external enemies. Said orifices 2a are located above the water level that may accumulate between the container capsule "b" and the top part 1, thanks to the proximity of the drainage channel 1c for extreme rains.

The feeding support or partition wall 2c can take other forms and allows food with a certain viscosity that stay on its place due to their shape or support rugosity. If manufactured by 3D printing of fused deposition modeling (FDM) it acquires said rugosity due to the production method. If manufactured by other methods, for example, by injection, the mold part for the partition wall requires a certain texture to transfer it to the piece.

The upper grooved body 2 of the container capsule "b" is manufactured with as many exit orifices as necessary, provided that the capsule permeability is not compromised. Drainages 1c of top part 1 are located on the inner face 1' of top part 1, in the area where the exit orifices 2a are located, to ensure water does not enter there.

In the cross-section of figure 4 it can be clearly seen the elongated central channel 2b that will receive the tree branch, and the exit orifices 2a of the beneficial insects. A partition wall 2c is defined under the elongated central channel 2b. Said partition wall 2c supports the position of the grid 3 and, due to its rugosity, can contain feeding solutions for the beneficial insects, for example, honey.

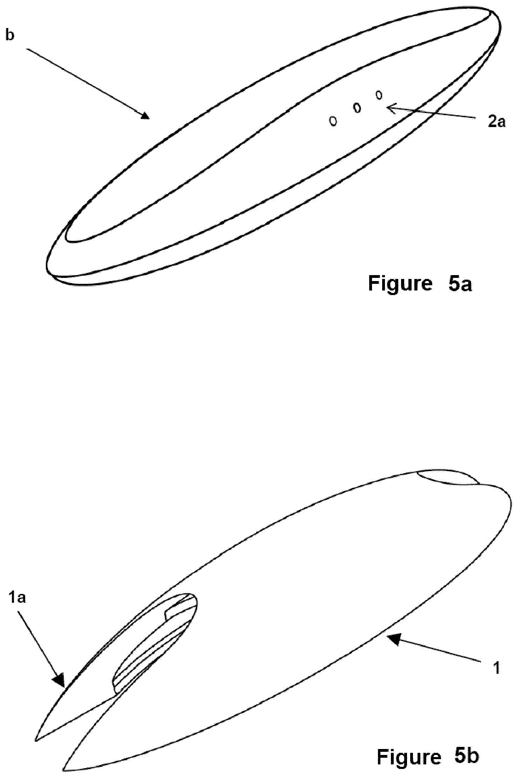

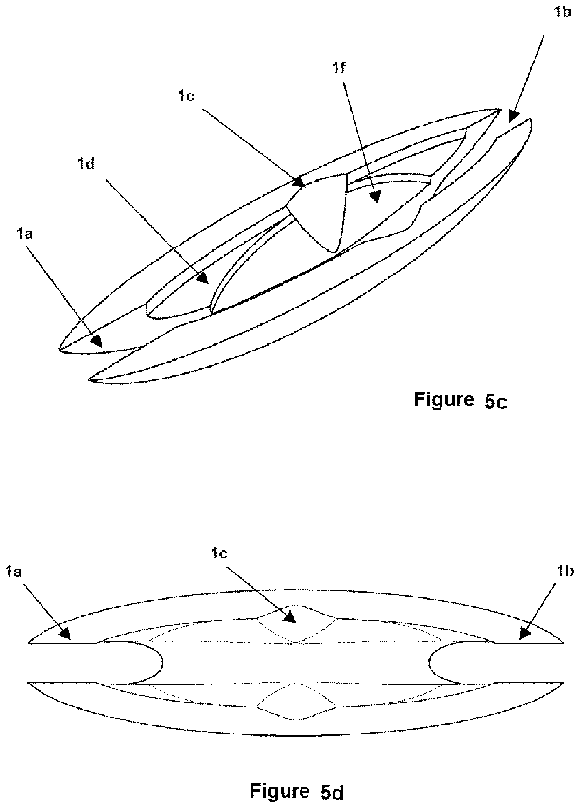

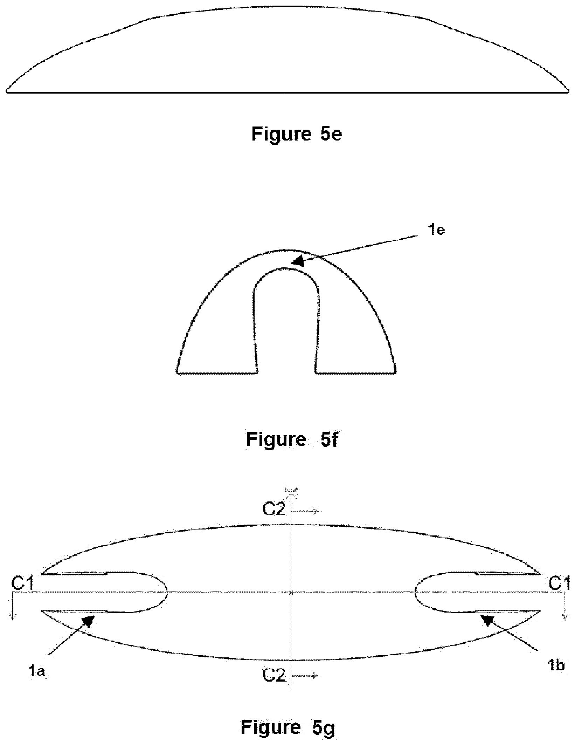

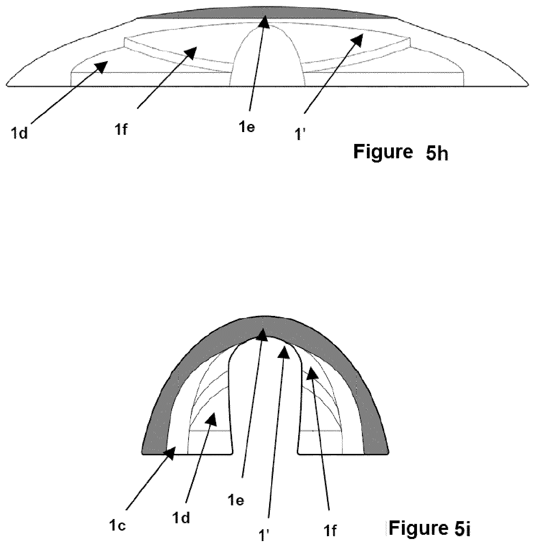

In Figures 5b, 5c, 5h and 5i it can be seen the ovoid structure of the top part 1, with its lower concave face 1' and its internal facets 1c, 1d, 1e and 1f; being 1c the extra drainage channel for extreme rain, 1d is the opposite shape of the container capsule in order to engage it, 1e is the narrow section at the top that allows the top part to flex and open their sides to receive the capsule, and finally 1f is the cavity that generates a clearance in the upward direction so that there is enough space between the exit orifices 2a and the top part 1 for the beneficial insects to come out.

In Figure 6g it can be seen how the lower hollow body 4, which contains the eggs of beneficial insects, decreases in inner width from the level of the line 4e to its upper inner peripheral edge 4d, which allows the grid 3 to be inserted by bending, and when stretched it gets trapped under said perimeter edge, thus remaining within the lower hollow body 4 and unable to be removed except by the use of a tool to take it from their orifices 3a. To better understand, in figure 4 it can be seen how said grid 3 gets contained due to the shape of the lower hollow body 4.

Coupling between the top part 1 and container capsule "b" is preferably achieved by flexing the lateral edges of the top part 1, which allows the container capsule "b" to be introduced between the lateral walls 1d of the top part 1, to finally produce a contraction that holds it tight in place.

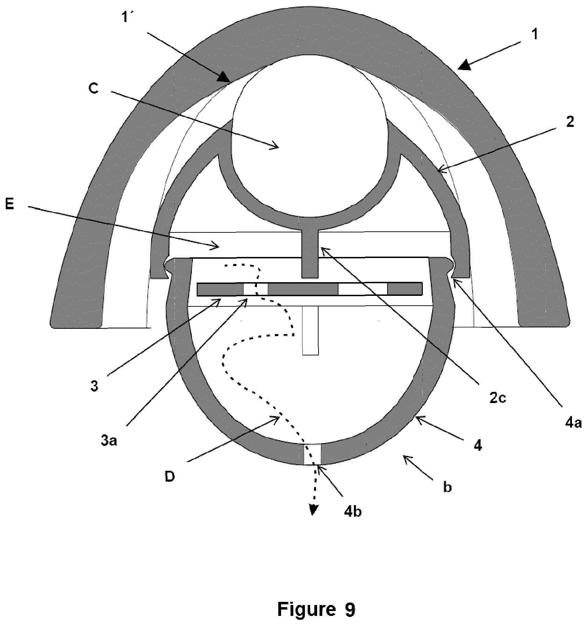

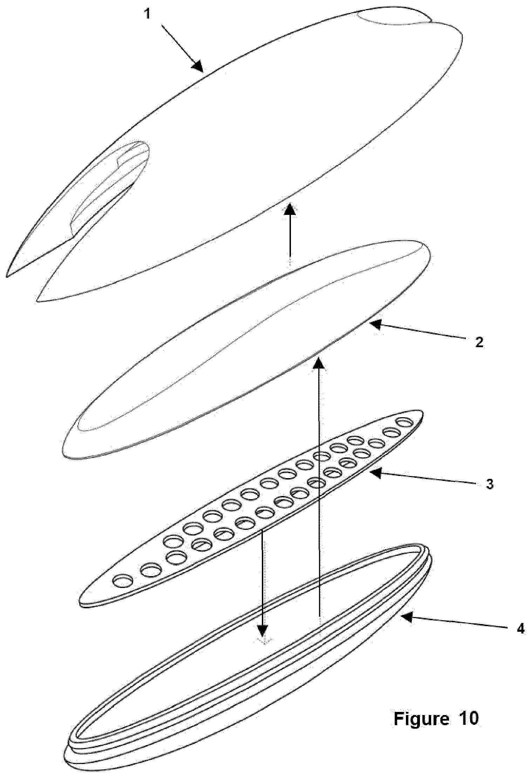

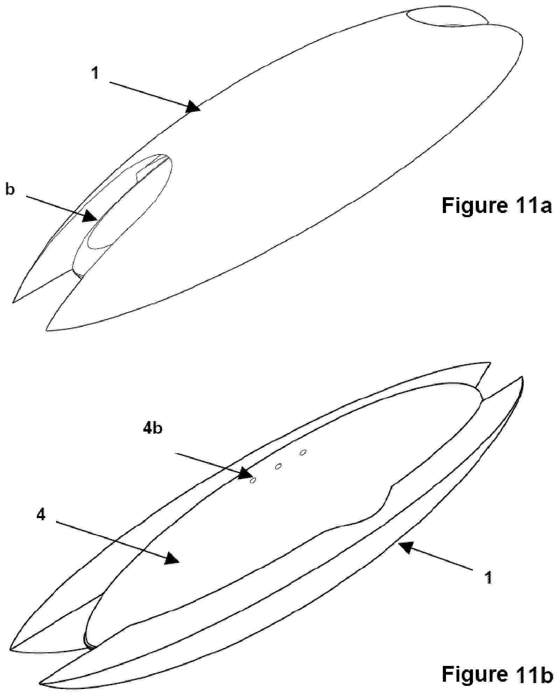

In figures 9 to 14 it can be seen a first variant of the biological pest control device which enables working with beneficial insects with positive or neutral geotropism, particularly, it can be seen that the exit orifices 2a of the first embodiment are no longer located in the upper part over the sides of the upper grooved body 2, instead, the exit orifices 4b for beneficial insects are now located under the lower hollow body 4 of the container capsule "b". It can be particularly seen in figure 9 the path "D" which would be traversed by the beneficial insects in this variant, that is, from chamber "E" to come out from under the lower hollow body 4 of the container capsule "b".

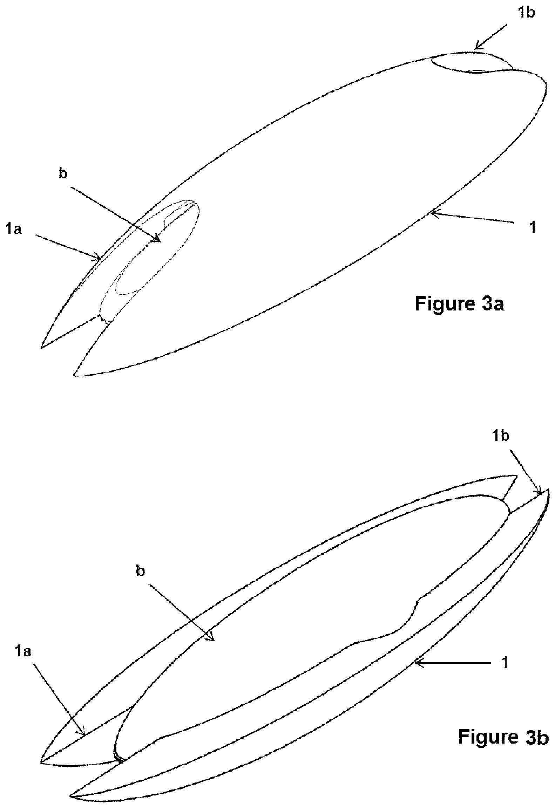

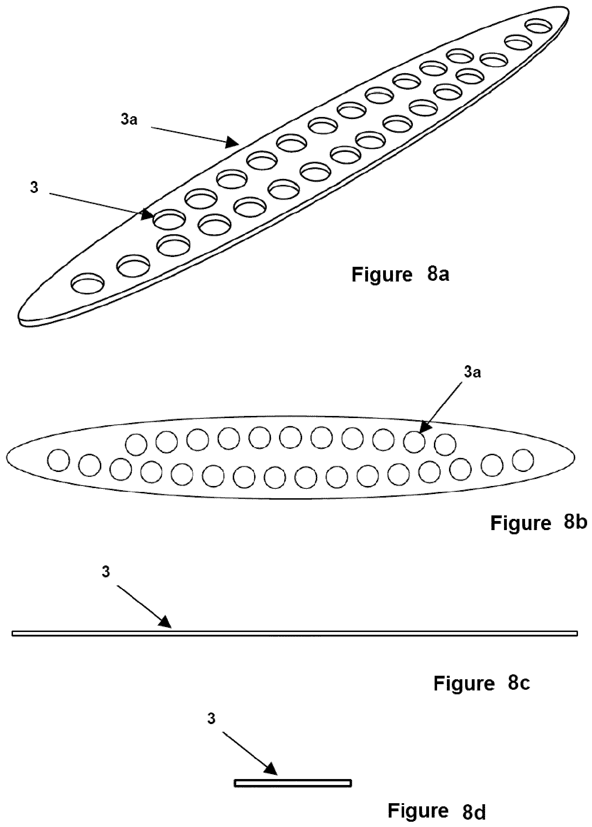

In this variant, the upper grooved body 2 does not serve as anteroom of the exit or feeding zone like in the first embodiment of figures 1 to 8, but serves as a container chamber. A few eggs are located in space "E" and, given that they have positive geotropism, they will try to go downwards after hatching and will come out through orifices 4b of the lower hollow body 4.

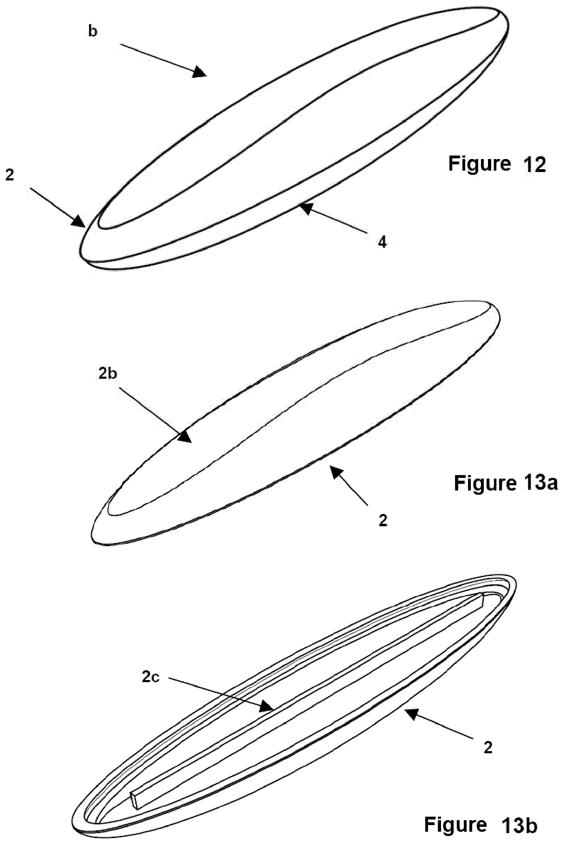

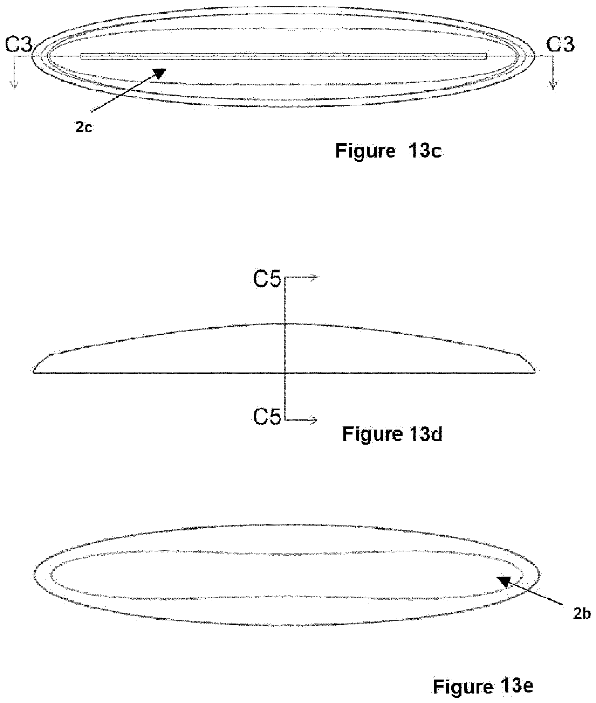

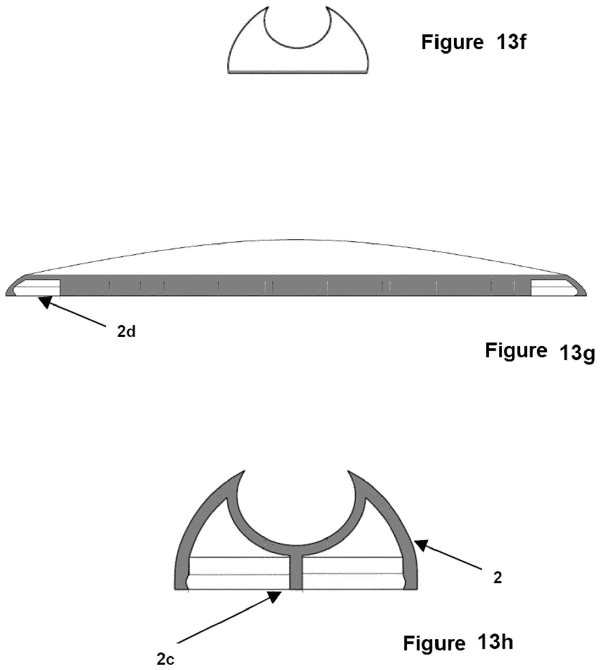

Particularly in figure 13g, it can be seen the interruption of the long partition wall 2d to allow for coupling between the edges of the upper grooved body 2 and the lower hollow body 4.

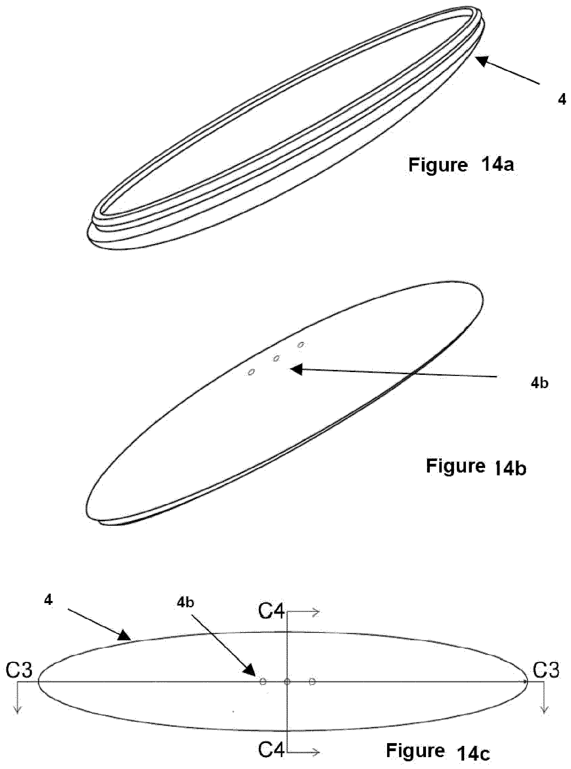

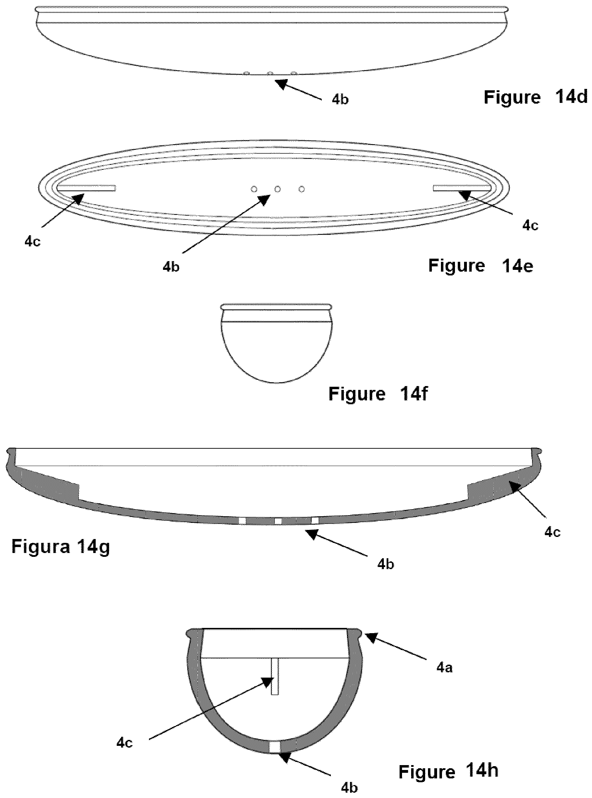

In figures 14g and 14h it can be seen the lower hollow body 4 of the variant embodiment of figure 9 with inner partition walls 4c on each of its ends, in order to enable placing of food for the beneficial insects that come downwards from chamber "E". Said partition walls 4c can take other forms to hold the food. In case of using sticky food, these partition walls 4c substitute for the use of partition wall 2c as a support means for food, given that the latter is adjacent chamber "E" which contains the eggs of the beneficial insects.

In all variants of embodiments of the present invention, figures 4, 9 and 15, it can be seen that the device features a wide cavity "C" to receive the tree branch, which allows, in case of rain, for the water to flow and so water does not accumulate, which also allows the circulation of animals through said space.

The contents of the capsule and he mass center of the top part are located below the branch, which ensures the stability of the device, given that its center of gravity is low. In addition to this, there is friction between the branch and the passageway of the device. If the branch is narrower than cavity "C", the device can still hold to it without rotating, because of the inherent irregularities in the branch direction that apply pressure in different points along cavity "C" of the device. In addition to this, smooth curves and aerodynamics of the exterior forms of the device reduce the effect that the winds can exert over the device. Because of that, the device, once installed, will remain on its place and correctly oriented.

If 3D printing is used for manufacturing the pieces, the thickness of the pieces can be thin, which reduces printing times, but has to be enough to ensure impermeability of the content and enough opacity to prevent inner heating because of a greenhouse effect. Said opacity is also selected in order to manipulate insects with marked phototropism.

In the variant where the device releases beneficial insects, figures 1 to 14, the device presents more efficiency and efficacy than other suspended devices with vulnerable beneficial insects, because the content is not affected by winds or extreme rain, even if it rains obliquely or horizontally, since the passageway formed between the container capsule "b" and the top part 1 is high enough so that when it rains the water flows through the branch inserted therein, and does not accumulate where device is installed, therefore it can be used not only inside greenhouses but also on the outside. The top part 1 has additionally an extra drainage channel for extreme rains 1c, which ensures that water does not accumulate where the orifices 2a are located.

Additionally, there will be an increased stability because the gravity center is lower than the branch, because the container is located on the inner part, which is also the heavier part of the top part, which enables to keep a correct orientation of the device.

Moreover, it has an exit 2a, figure 4 or 4b, figure 9, next to the branch that allows the beneficial insects to travel toward the affected plant without obstacles, climbing suspending systems, such as hooks or cords, flying or jumping because they cannot find the way to the affected zone.

The parts of the device can be adapted according to the characteristics of the beneficial insect, the environment and any other determining fact of the strategy. For example, the location and diameter of the exit orifices are determined to enable the beneficial insects to come out, and the passageway width for the branch is defined according to the branch diameter where the device is to be installed, which in forest plantations in the agricultural industry tends to be similar because they are the same age.

The container chamber will contain beneficial insects in a mobile state or eggs of the beneficial insects. Said eggs can be place by stacking or over leaf cuttings, which enables for easier handling and prevents them from passing through the orifices 3a of the grid 3.

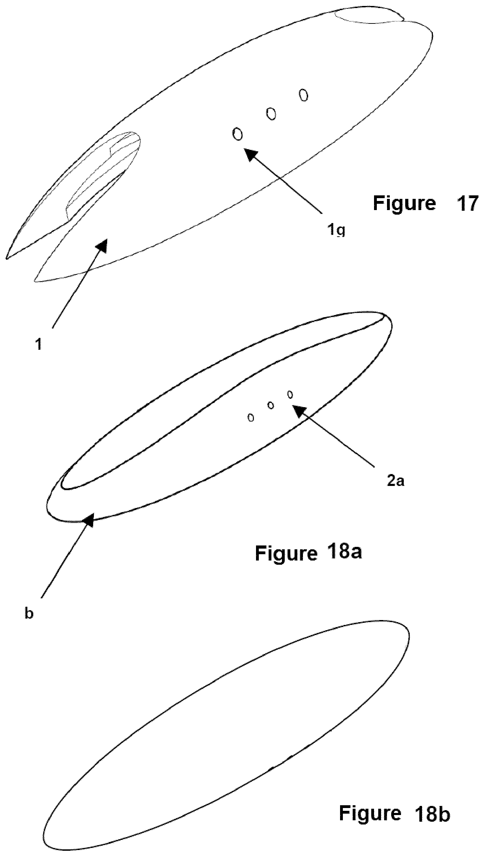

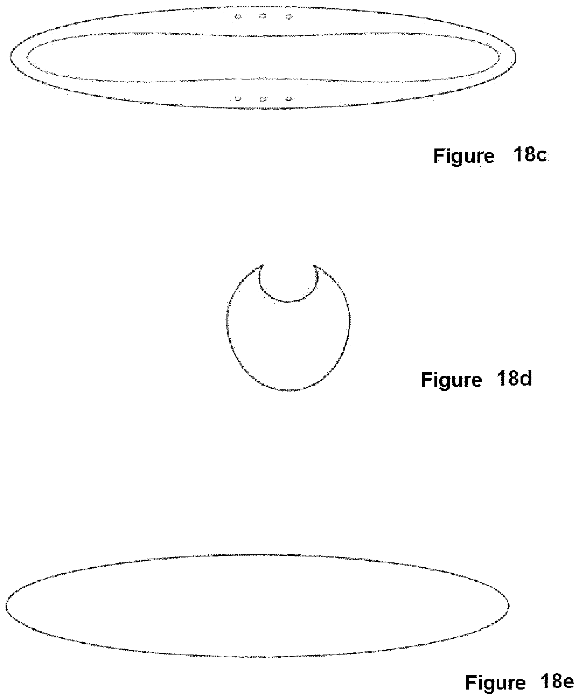

If the device is to be used as a trap or repellent, the joint between parts of the capsule will be hermetic or the capsule must be manufactured as a single piece (see figures 18a to 18e) because it will house some form of liquid and/or substances. In this case, exit orifices 2a (figure 15) should be sealed so the content does not get spilt during transportation, for example, with and adhesive tape that covers them or plugs occluding them.

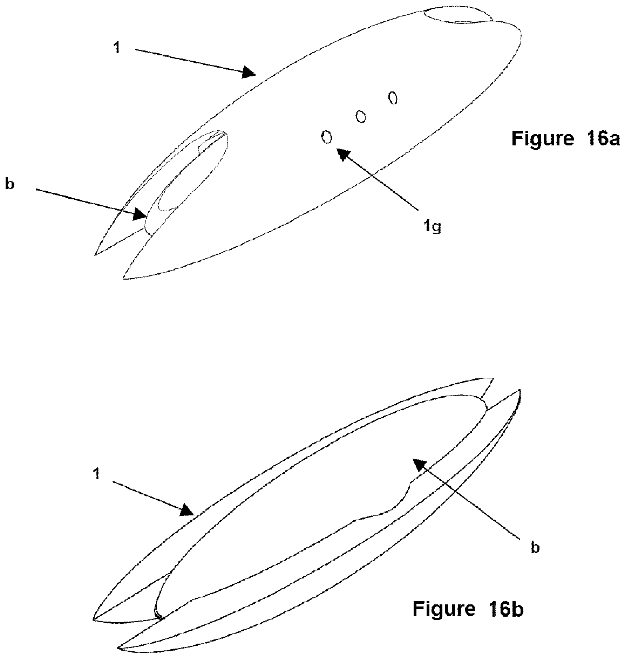

When used as a trap, as illustrated in figure 15, the device comprises a top part 1 to which orifice 1g (see also figure 16a) are made so scents or toxic substances can spread and flying pests that arrive directly through the top part can access to these substances. In the case of flying pests or pests that do not come along the branch, they will access through said orifices or passageways 1g which communicate the outside with the cavity formed between the top part 1 and the container capsule "b".

The container capsule "b" will contain an attracting substance "X" and/or toxic substances, for example, a liquid, that the pest will access either from the branch, after going through the orifices 2a made on the upper face of said container capsule "b" or from the top part 1 after going through orifices 1g.

Finally, a variant as a deterrent device is like the repellent device, except that shapes (such as in figure 20) and/or colors are selected to repel the pest to reinforce the repellent scent. Customization of different shapes 3D printing, by producing low numbers for experimentation and applications for different species, is inexpensive and enables the selection between choosing a mimetic device that gets lost in the environment, which causes confusion to the pest, or highlighting the device to be able to find it easily or to catch the attention of pests.

Since in this variant it is not necessary to achieve free movement of animals along the branch, the control device can omit the space between the upper convex side surface of the container capsule "b" and the inner surface 1f of the top part 1, leaving only space for the tree branch, so it may have decreased thickness and height than those of the previous variants, which reduces costs of materials and production times.

## Claims

1. A biological pest control device which comprises a top part (1) defining grooves in its ends (1a, 1b), said top part being coupled over a container capsule (b) with beneficial insects or substances that help to control pests, wherein the top part (1) has a lower face (1') substantially concave and on the upper face of the container capsule (b) there is formed a concavity (2b) thereby defining between the lower face (1') of the top part (1) and the external upper surface of the container capsule (b) a cavity (C), extending from side to side of the device, which is capable of housing a linear element to suspend the biological pest control device; wherein the top part (1) has extra drainage channels (1c) for extreme rains, located in the inner faces of the top part (1), and the container capsule has an exit facing an inner face of the top part, said exit being above the level to which water can get accumulated, between a capsule and the top part when water flows along the linear element that goes through the device from side to side, so that water does not enter the capsule.

2. The biological pest control device according to claims 1, wherein the container capsule (b) comprises: a lower hollow body (4) that serves as a container for beneficial insects or eggs of beneficial insects with negative or neutral geotropism, a grid (3) with orifices (3a) that covers said lower hollow body (4); and an upper groove body (2) with orifices (2a) which couples to said lower hollow body (4) in perimeter edges; and wherein there is a partition wall (2c) under said upper grooved body (2) that is capable of holding food for the beneficial insects.

3. The biological pest control device according to claim 1, wherein the container capsule (b) comprises: a lower hollow body (4) that serves as an anteroom of the exit for beneficial insects with positive or neutral geotropism, a grid (3) with orifices (3a) that covers said lower hollow body (4) with exit orifices for beneficial insects (4b); and an upper groove body (2) upon which there is defined a concave surface (2b); said body couples to said lower hollow body (4); and wherein there is a partition wall (2c) under the upper grooved body (2) that is capable of holding food for the beneficial insects.

4. A biological pest control device according to claim 3, wherein the lower hollow body (4) comprises inner partition walls (4c) capable of holding food.

5. A biological pest control device according to claim 1, wherein the container capsule (b) comprises upper exit orifices (2a) located over the substances that are capable of repelling or attracting pests that the capsule contains; and the top part (1) comprises orifices (1g).

6. A biological pest control device according to any one of claims 1 to 5, wherein the top part (1) has an ovoid structure.

7. A biological pest control device according to any one of claims 2 or 3, wherein the grid (3) is made of a flexible material and can be secured by flexing pressure inside the lower hollow body (4), given that it has a larger inner length than its perimeter edges (4d).

8. A biological pest control device according to any one of claims 2 or 3, wherein the partition wall (2c) is rectangular and traverses along the upper grooved body (2) in which it is defined.

## Patentansprüche

1. Biologische Schädlingsbekämpfungsvorrichtung, die einen Oberteil (1) umfasst, der in seinen Enden (1a, 1b) Rillen definiert, wobei der Oberteil über eine Behälterkapsel (b) mit Nützlingen oder Substanzen gekoppelt ist, die bei der Bekämpfung von Schädlingen helfen, wobei der Oberteil (1) eine untere Fläche (1') aufweist, die im Wesentlichen konkav ist, und auf der oberen Fläche der Behälterkapsel (b) eine Konkavität (2b) ausgebildet ist, wodurch zwischen der unteren Fläche (1') des Oberteils (1) und der äußeren oberen Fläche der Behälterkapsel (b) ein Hohlraum (C) definiert wird, der sich von einer Seite zur anderen Seite der Vorrichtung erstreckt und der in der Lage ist, ein lineares Element zum Aufhängen der biologischen Schädlingsbekämpfungsvorrichtung aufzunehmen; wobei der Oberteil (1) zusätzliche Drainagekanäle (1c) für extreme Regenfälle aufweist, die in den Innenflächen des Oberteils (1) angeordnet sind, und die Behälterkapsel einen Ausgang aufweist, der einer Innenfläche des Oberteils zugewandt ist, wobei der Ausgang oberhalb des Niveaus liegt, bis zu dem sich Wasser zwischen einer Kapsel und dem Oberteil ansammeln kann, wenn Wasser entlang des linearen Elements fließt, das von Seite zu Seite durch die Vorrichtung geht, so dass Wasser nicht in die Kapsel eintritt.

2. Biologische Schädlingsbekämpfungsvorrichtung nach Anspruch 1, wobei die Behälterkapsel (b) umfasst: einen unteren Hohlkörper (4), der als Behälter für Nützlinge oder Eier von Nützlingen mit negativem oder neutralem Geotropismus dient, ein Gitter (3) mit Öffnungen (3a), das den unteren Hohlkörper (4) bedeckt; und einen oberen Rillenkörper (2) mit Öffnungen (2a), der an den unteren Hohlkörper (4) in Umfangsrändern anschließt; und wobei unter dem oberen Rillenkörper (2) eine Trennwand (2c) vorhanden ist, die in der Lage ist, Nahrung für die Nützlinge zu halten.

3. Biologische Schädlingsbekämpfungsvorrichtung nach Anspruch 1, wobei die Behälterkapsel (b) umfasst: einen unteren Hohlkörper (4), der als Vorraum des Ausgangs für Nützlinge mit positivem oder neutralem Geotropismus dient, ein Gitter (3) mit Öffnungen (3a), das den unteren Hohlkörper (4) mit Ausgangsöffnungen für Nützlinge (4b) bedeckt und einen oberen Rillenkörper (2), auf dem eine konkave Oberfläche (2b) definiert ist; wobei der Körper mit dem unteren Hohlkörper (4) gekoppelt ist; und wobei es eine Trennwand (2c) unter dem oberen Rillenkörper (2) gibt, die in der Lage ist, Nahrung für die Nützlinge zu halten.

4. Biologische Schädlingsbekämpfungsvorrichtung nach Anspruch 3, wobei der untere Hohlkörper (4) innere Trennwände (4c) aufweist, die in der Lage sind, Nahrung zu halten.

5. Biologische Schädlingsbekämpfungsvorrichtung nach Anspruch 1, wobei die Behälterkapsel (b) obere Ausgangsöffnungen (2a) umfasst, die sich über den Substanzen befinden, die in der Lage sind, Schädlinge, die die Kapsel enthält, abzustoßen oder anzuziehen; und der Oberteil (1) Öffnungen (1g) umfasst.

6. Biologische Schädlingsbekämpfungsvorrichtung nach einem der Ansprüche 1 bis 5, wobei der Oberteil (1) eine eiförmige Struktur aufweist.

7. Biologische Schädlingsbekämpfungsvorrichtung nach einem der Ansprüche 2 oder 3, wobei das Gitter (3) aus einem flexiblen Material besteht und durch Biegedruck im Inneren des unteren Hohlkörpers (4) befestigt werden kann, angesichts der Tatsache, dass es eine größere Innenlänge als seine Umfangsränder (4d) aufweist.

8. Biologische Schädlingsbekämpfungsvorrichtung nach einem der Ansprüche 2 oder 3, wobei die Trennwand (2c) rechteckig ist und entlang des oberen Rillenkörpers (2), in dem sie definiert ist, quert.

## Revendications

1. Dispositif de lutte biologique contre les nuisibles, qui comprend une partie supérieure (1) définissant des rainures dans ses extrémités (1a, 1b), ladite partie supérieure étant couplée sur une capsule conteneur (b) avec des insectes bénéfiques ou des substances qui aident à lutter contre les nuisibles, dans lequel la partie supérieure (1) a une face inférieure (1') substantiellement concave et sur la face supérieure de la capsule conteneur (b) est formée une concavité (2b) définissant ainsi entre la face inférieure (1') de la partie supérieure (1) et la surface supérieure externe de la capsule conteneur (b) une cavité (C), s'étendant d'un côté à l'autre du dispositif, qui est capable de loger un élément linéaire pour suspendre le dispositif de lutte biologique contre les nuisibles ; dans lequel la partie supérieure (1) comporte des canaux de drainage supplémentaires (1c) pour les pluies extrêmes, situés dans les faces internes de la partie supérieure (1), et la capsule conteneur comprend une sortie qui fait face à une face intérieure de la partie supérieure, ladite sortie étant au-dessus du niveau jusqu'auquel de l'eau peut être accumulée, entre une capsule et la partie supérieure lorsque l'eau s'écoule le long de l'élément linéaire qui traverse le dispositif d'un côté à l'autre, de telle sorte que l'eau ne pénètre pas dans la capsule.

2. Dispositif de lutte biologique contre les nuisibles selon la revendication 1, dans lequel la capsule conteneur (b) comprend : un corps creux inférieur (4) qui sert de récipient pour des insectes utiles ou des œufs d'insectes utiles ayant un géotropisme négatif ou neutre, une grille (3) avec des orifices (3a) qui recouvrent ledit corps creux inférieur (4) ; et un corps rainuré supérieur (2) avec des orifices (2a) qui se couple audit corps creux inférieur (4) au niveau des bords du périmètre ; et dans lequel il y a une paroi de séparation (2c) sous ledit corps rainuré supérieur (2) qui est capable de contenir de la nourriture pour les insectes utiles.

3. Dispositif de lutte biologique contre les nuisibles selon la revendication 1, dans lequel la capsule conteneur (b) comprend : un corps creux inférieur (4) qui sert d'antichambre de sortie pour les insectes utiles à géotropisme positif ou neutre, une grille (3) avec des orifices (3a) qui recouvre ledit corps creux inférieur (4) avec des orifices de sortie pour les insectes utiles (4b) ; et un corps rainuré supérieur (2) sur lequel est définie une surface concave (2b) ; ledit corps s'accouple audit corps creux inférieur (4) ; et dans lequel il y a une paroi de séparation (2c) sous le corps rainuré supérieur (2) qui est capable de contenir de la nourriture pour les insectes utiles.

4. Dispositif de lutte biologique contre les nuisibles selon la revendication 3, dans lequel le corps creux inférieur (4) comprend des parois de séparation intérieures (4c) capables de contenir de la nourriture.

5. Dispositif de lutte biologique contre les nuisibles selon la revendication 1, dans lequel la capsule conteneur (b) comprend des orifices de sortie supérieurs (2a) situés au-dessus des substances qui sont capables de repousser ou d'attirer les nuisibles que la capsule contient ; et la partie supérieure (1) comprend des orifices (1g).

6. Dispositif de lutte biologique contre les nuisibles selon l'une quelconque des revendications 1 à 5, dans lequel la partie supérieure (1) présente une structure ovoïde.

7. Dispositif de lutte biologique contre les nuisibles selon l'une quelconque des revendications 2 ou 3, dans lequel la grille (3) est faite d'un matériau flexible et peut être fixée par pression de flexion à l'intérieur du corps creux inférieur (4), étant donné qu'elle a une longueur intérieure plus grande que ses bords périphériques (4d).

8. Dispositif de lutte biologique contre les nuisibles selon l'une quelconque des revendications 2 ou 3, dans lequel la paroi de séparation (2c) est rectangulaire et traverse le long du corps supérieur rainuré (2) dans lequel elle est définie.
